# EUROPEAN PATENT APPLICATION

(11) **EP 4 789 609 A1**
(43) Date of publication of application: **12.08.2026**
(21) Application number: 26155453.9
(22) Date of filing: 30.01.2026
(51) Int. Cl.: A61B 5/01, A61B 5/145, A61B 5/1459, A61B 5/1473, A61B 5/1495, A61B 5/00

(54) **DETERMINING INTRAVASCULAR PCO2**

(30) Priority: 07.02.2025 SE 2550129
(71) Applicant: NeoSense Technologies AB, 169 70 Solna (SE)
(72) Inventor: DANEHORN, Kenneth, 185 51 Vaxholm (SE)
(74) Representative: Barker Brettell Sweden AB

(57) **Abstract**

A system (1) for determining intravascular partial pressure of carbon dioxide (pCO₂) comprises an intravascular oxygen tension (pO₂) sensor (10) configured to measure pO₂ in a subject, an oxygen saturation (SO₂) sensor (20) configured to measure sO₂ in the subject, an intravascular pH sensor (30) configured to measure pH in blood of the subject and a temperature sensor (40) configured to measure temperature of the subject. The system (1) also comprises a processor (50) and a memory (60) coupled to the processor (50) and comprising instructions executable by the processor (50) to cause the processor (50) to determine intravascular pCO₂ for the subject based on the measured pO₂, SO₂, pH and temperature.

## Description

### TECHNICAL FIELD

The present invention generally relates to a system for determining intravascular partial pressure of carbon dioxide (pCO₂).

### BACKGROUND

The carbon dioxide tension or partial pressure of carbon dioxide (pCO₂) is a vital parameter when monitoring critically ill patients. Normal values of pCO₂ in human blood are in the range from 4.7 up to 6.0 kPa. Higher values of pCO₂ than normal is referred to as hypercapnia and is generally caused by hypoventilation, lung disease, hypothermia, metabolic disorders, or brain damage. A state of hypercapnia can also be an intentional clinical strategy for patients exposed to mechanical ventilation to minimize lung injury caused by high tidal volumes and high airway pressure. Hypercapnia can have harmful effects, such as impairment of alveolar function, cell proliferation, and muscle function. Acute hypercapnia may have significant hemodynamic consequences that can lead to pulmonary hypertension, right ventricular dysfunction, or prolonged bronchopleural leakage.

Lower values of pCO₂ than normal is referred to as hypocapnia and is caused by hyperventilation, which increases the diffusion gradient of CO₂ from venous blood to the alveoli resulting in a net removal of CO₂ from the body. Many pathological causes can generate hyperventilation and result in hypocapnia including, but not limited to, asthma, bacterial sepsis, pneumonia, head trauma, meningitis, metabolic acidosis, panic disorder, pneumothorax, pulmonary edema, and pulmonary embolism. Hypocapnia is, however, well tolerated by patients and no major complications are due only to low pCO₂. A low level of pCO₂ is, however, an indicator for mechanically ventilated patients that the ventilated minute volume is too high.

There are few acceptable technologies for measuring intravascular pCO₂, i.e., pCO₂ in blood. The golden standard is to measure pCO₂ from a blood sample by means of an advanced electrolytic cell in a Blood Gas Analyzer (BGA). The electrolytic cell contains a membrane that allows only uncharged molecules of CO₂, O₂, and N₂ to pass through the membrane. Dissolved CO₂ will affect the pH-value in the electrolyte, which is used as an indirect measurement of pCO₂. This technology is problematic to implement in an *in vivo* environment due to the need for the CO₂ penetrable membranes, why a continuous breath-by-breath end-tidal (P_{ET,CO2}) measurement may be used as a substitute for intravascular measurements of pCO₂. P_{ET,CO2} is commonly measured by capnographs using infrared light sensors. Intravascular pCO₂ can also be estimated by means of a transcutaneous measurement using a heated skin electrode. The transcutaneous CO₂ monitoring can only be used intermittently and still suffers from unreliability.

US 2010/0057046 discloses a system for continuously measuring a physiologic parameter of a patient. The system includes a probe having an elongate body and configured to be inserted into a location within a patient. At least one sensor can be operably connected to the probe and configured to continuously provide real-time feedback information on one or more physiologic parameters at the location within the patient, such as pH, pCO₂, pO₂, pressure, or temperature. A controller is connected to the probe and configured to receive the real-time feedback information and to adjust a therapeutic setting on a therapeutic device based at least in part on the feedback information.

US 2021/0282680 discloses a catheter configured to detect at least one blood gas parameter present in blood in a blood-vessel of a patient. The catheter has a catheter wall forming a lumen configured for umbilical arterial catheterization. At least one optical fiber is incorporated in the catheter wall and is configured to detect at least one blood gas parameter.

### SUMMARY

It is a general objective to provide a system capable of determining intravascular pCO₂ in a subject.

This and other objectives are met by the embodiments.

The present invention is defined in the independent claim. Further embodiments are defined in the dependent claims.

An aspect of the invention relates to a system for determining intravascular pCO₂. The system comprises an intravascular oxygen tension (pO₂) sensor configured to measure pO₂ in a subject, an oxygen saturation (SO₂) sensor configured to measure SO₂ in the subject, an intravascular pH sensor configured to measure pH in blood of the subject and a temperature sensor configured to measure temperature of the subject. The system also comprises a processor and a memory coupled to the processor and comprising instructions executable by the processor to cause the processor to determine intravascular pCO₂ for the subject based on the measured pO₂, SO₂, pH and temperature.

The present invention determines intravascular pCO₂ by processing a plurality of sensor readings rather than using a state-of-the-art electrolytic cell and specially designed CO₂ transparent membranes to measure pH value as an indirect measurement of pCO₂. An accurate pCO₂ determination is thereby achieved with robust measurement techniques and relaxing the needs for CO₂ transparent membranes having complications in *in vivo* environment.

### BRIEF DESCRIPTION OF THE DRAWINGS

The embodiments, together with further objects and advantages thereof, may best be understood by making reference to the following description taken together with the accompanying drawings, in which:
FIG. 1: The figure illustrates an intravascular catheter comprising an electrochemical pO₂ sensor and a temperature sensor. The pO₂ sensor is realized by two ring electrodes integrated on the catheter tube and the temperature sensor is realized by a thermocouple integrated into a catheter lumen according to an embodiment. The ring electrodes and thermocouple are connected to electronic units through connecting wires that run through a catheter lumen to a contact at the proximal end of the catheter.
FIG. 2: The figure illustrates an intravascular catheter for invasive measurement of SO₂ according to an embodiment. The intravascular catheter comprises two optical fibers running through a catheter lumen in the intravascular catheter with an efferent optical fiber illuminating circulating blood at the distal end, and an afferent optical fiber measuring reflected light from the red blood cells.
FIG. 3: The figure illustrates an intravascular catheter for invasive measurement of pH value in blood according to an embodiment. The pH sensor comprises an ISFET transistor positioned in a catheter lumen where an opening in the catheter tube enables the pH sensor to be in direct contact with blood. A reference electrode is in contact with the patient and can be in the form of a patch attached to the skin or an electrode on the intravascular catheter.
FIG. 4: The figure illustrates a system for determining pCO₂ according to an embodiment.
FIG. 5: The figure illustrates (A) an intravascular catheter according to an embodiment for measurement of pO₂, temperature, SO₂, and pH value and (B) a cross-sectional view of the intravascular catheter.
FIG. 6: The figure illustrates cross sections of two intravascular catheters according to an embodiment. A venous catheter is used for measurement of body temperature and pH and an arterial catheter is used for pO₂ and SO₂ measurement.
FIG. 7: The figure illustrates a graph showing pCO₂ as determined by the system of the invention (Estimated pCO₂), as determined using the gas mix to the oxygenator in an ECMO system (Gas mix pCO₂), and as measurements by a blood gas analyzer (Radiometer 800 Flex).
FIG. 8: The figure illustrates the relationship between SO₂ and pO₂ and the shift in the relationship depending on temperature, pH-value and CO₂.
FIG. 9: The figure illustrates (A) the distribution of oxygen saturation and oxygen tension values at normal blood values and (B) a sigmoid curve fitted to the distribution of oxygen saturation and oxygen tension values.
FIG. 10: The figure illustrates spectra corresponding to 98 % oxygen saturation and 57 % oxygen saturation.
FIG. 11: The figure is a flow chart illustrating a method for determining partial pressure of carbon dioxide in blood.

### DETAILED DESCRIPTION

The present invention generally relates to a system for determining intravascular partial pressure of carbon dioxide (pCO₂).

There is generally a need in healthcare facilities to get information of the blood concentration of carbon dioxide, particularly at intensive care units. Such blood concentration of carbon dioxide is usually represented by the partial pressure of carbon dioxide (pCO₂) in the blood, also referred to as carbon dioxide tension. In intensive care units, the level of pCO₂ is controlled by settings of respiratory rate and tidal volume in mechanically ventilated patients. For instance, a low level of pCO₂ is an indicator for such mechanically ventilated patients that the ventilated minute volume is too high, whereas a higher level of pCO₂ could be desired for patients exposed to mechanical ventilation to minimize lung injury caused by high tidal volumes and high airway pressure.

pCO₂ is also valuable information when diagnosing patients with diseases that cause disturbances to a normal pCO₂ level. For instance, various lung diseases may cause high pCO₂ levels, whereas several conditions can cause low pCO₂ levels including, but not limited, to asthma, bacterial sepsis, pneumonia, head trauma, meningitis, metabolic acidosis, panic disorder, pneumothorax, pulmonary edema, and pulmonary embolism.

An elevated level of pCO₂ is associated with higher risk for several complications at intensive care for adult, pediatric, and neonatal patients. There is, therefore, a need for a technology that enables determination of intravascular pCO₂, preferably estimation of intravascular pCO₂ continuously in real time.

Intravascular pCO₂ as referred to herein corresponds to the pCO₂ in blood of a subject. The intravascular pCO₂ is preferably arterial pCO₂, i.e., pCO₂ determined for arterial blood of the subject. Alternatively, the intravascular pCO₂ could be venous pCO₂, i.e., pCO₂ determined for venous blood of the subject.

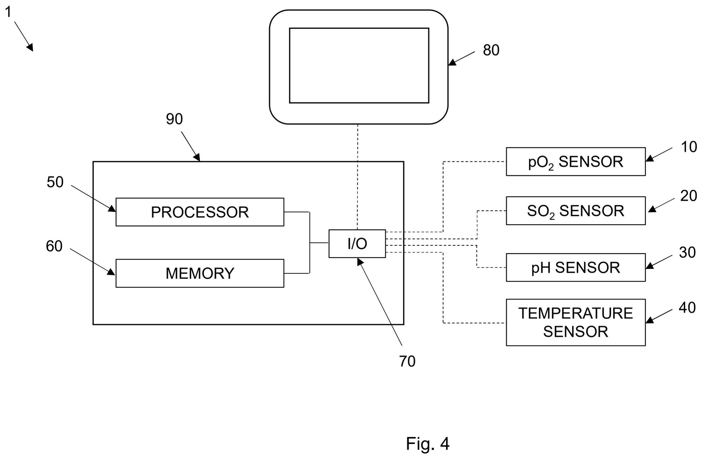

An aspect of the invention relates to a system 1 for determining intravascular pCO₂, see Fig. 4. The system 1 comprises an intravascular oxygen tension (pO₂) sensor 10 configured to measure pO₂ in a subject. The system 1 also comprises an oxygen saturation (SO₂) sensor 20 configured to measure SO₂ in the subject. The system 1 further comprises an intravascular pH sensor 30 configured measure pH in blood of the subject. The system 1 additionally comprises a temperature sensor 40 configured to measure temperature of the subject. The system 1 also comprises a processor 50 and a memory 60 coupled to the processor 50 and comprising instructions executable by the processor 50 to cause the processor 50 to determine intravascular pCO₂ for the subject based on the measured pO₂, SO₂, pH and temperature.

The system 1 of the invention, thus, uses different parameters as measured for a given subject, i.e., pO₂, SO₂, pH and temperature. The values as determined for these parameters are processed by the processor 50 to determine or calculate a value of intravascular pCO₂ for the subject. This means that the processor 50 is caused when executing instructions stored in the memory 60 to determine the intravascular pCO₂ for the subject as a function f( ) of the measured pO₂, SO₂, pH and temperature, i.e., pCO2 = f(pO2, SO2, pH-value, temperature).

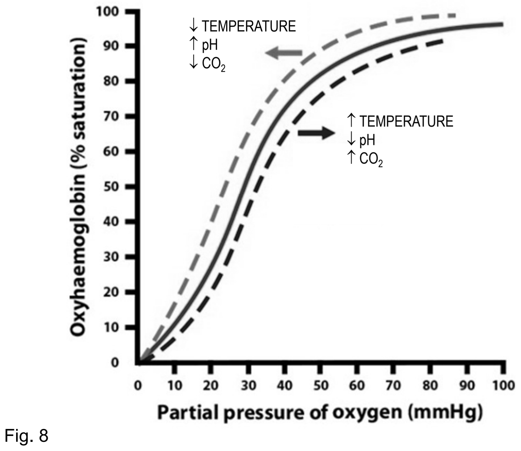

The relation between oxygen tension (pO₂) and oxygen saturation (SO₂) in human blood can be described as a sigmoid shaped curve, see Fig. 8, where pO₂ is mapped to the X-axis and SO₂ is mapped to the Y-axis. This relationship is commonly referred to as the oxygen dissociation curve (ODC) and is governed by the affinity of hemoglobin. Hemoglobin affinity is in turn affected by various properties of the blood, including temperature, pH and partial pressure of carbon dioxide. In other words, the ODC will shift to the right or left dependent on these blood parameters. In more detail, an increase in temperature, a decrease in pH and an increase in partial pressure of carbon dioxide in the blood all cause a right shift of the ODC as indicated in Fig. 8, whereas a decrease in temperature, an increase in pH and a decrease in partial pressure of carbon dioxide in the blood instead cause a left shift of the ODC. This means that the ODC will have a unique relationship between oxygen tension and oxygen saturation depending on the particular values of these properties of the blood. This dependency of the relationship between oxygen tension and oxygen saturation on blood temperature, pH and partial pressure of carbon dioxide is employed by the system 1 of the invention to derive the intravascular partial pressure of carbon dioxide based on measured intravascular oxygen tension, oxygen saturation, intravascular pH and temperature.

A significant advantage of the system 1 of the invention as compared to blood sampling and measuring pCO₂ in the blood sample using a BGA is that the system 1 can determine and monitor the intravascular pCO₂ in real-time by directly measuring the properties on the subject body without the need for any blood sampling and separate analysis in a BGA. The pCO₂ can, thus, be determined in real time by the system 1 based on real-time measurements of pO₂, SO₂, pH and temperature.

Hence, in an embodiment, the intravascular pO₂ sensor 10 is configured to measure pO₂ in real time in the subject and generate a real-time pO₂ estimate. The SO₂ sensor 20 is configured to measure SO₂ in real time in the subject and generate a real-time SO₂ estimate. In this embodiment, the intravascular pH sensor 30 is configured to measure pH in real time in blood of the subject and generate a real-time pH estimate and the temperature sensor 40 is configured to measure temperature in real time of the subject and generate a real-time temperature estimate. The memory 60 comprises, in this embodiment, instructions executable by the processor 50 to cause the processor 50 to determine a real-time intravascular pCO₂ estimate based on the real-time pO₂ estimate, the real-time SO₂ estimate, the real-time pH estimate and the real-time temperature estimate.

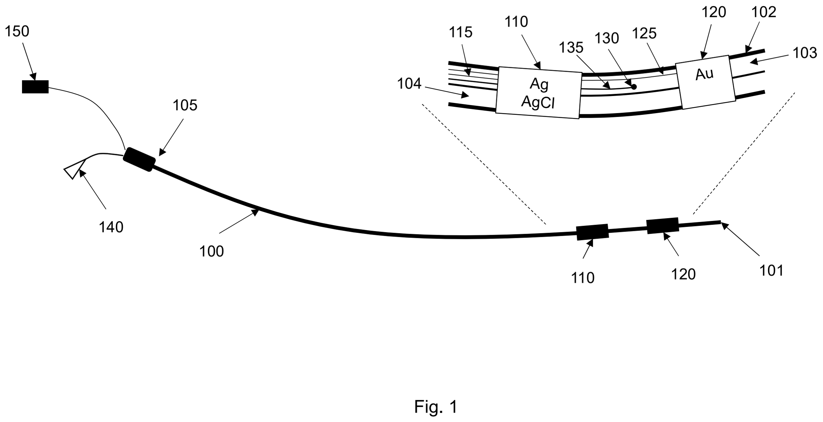

In an embodiment, the intravascular pO₂ sensor 10 is an intravascular electrochemical sensor 10 comprising, see Fig. 1, a working electrode 120 and a reference electrode 110 configured to be in contact with the blood the subject.

In an embodiment, the working electrode 120 is made of, or at least comprises a surface made of, a conductive material selected from the group consisting of gold, silver and carbon. In the latter carbon case, the surface of the working electrode 120 could be made of conductive pyrolityic glassy carbon or graphene as illustrative, but non-limiting, examples. A preferred conductive material is gold. Hence, in a preferred embodiment, the working electrode 120 comprises a surface made of gold or the working electrode 120 is made of solid gold.

In an embodiment, the reference electrode 110 is made of, or at least comprises a surface made of, Ag/AgCl, i.e., Ag and/or AgCl. This conductive material is capable of resisting the anodic load that the reference electrode 110 is exposed to during operation and maintains a stable potential at the intended use.

Only the surfaces or surface layers of the working electrode 120 and the reference electrode 110 are active in the electrochemical process. Accordingly, the electrodes 110, 120 do not need to be solid gold, silver or carbon material or solid Ag/AgCl material. This means that the active surface layer of gold, silver or carbon, or of Ag/AgCl could be deposited onto another suitable conductive material. Hence, in an embodiment the working electrode 120 is made of the conductive material selected from the group consisting of gold, silver and carbon, preferably gold. In this embodiment the working electrode 120 is made as a unitary structure of the selected conductive material. In another embodiment, the working electrode 120 is made of a conductive base or bulk material and has a surface layer, such as film or coating, deposited onto the conductive base or bulk material. In this embodiment, the surface layer is made of a conductive material selected from the group consisting of gold, silver and carbon, preferably gold. The conductive base or bulk material could then be made of a non-catalytic material, such as platinum or stainless steel. Correspondingly, in an embodiment the reference electrode 110 is made of Ag/AgCl. In this embodiment the reference electrode 110 is made as a unitary structure of Ag/AgCl. In another embodiment, the reference electrode 110 is made of a conductive base or bulk material and has a surface layer, such as film or coating, deposited onto the conductive base or bulk material. In this embodiment, the surface layer is made of Ag/AgCl. The conductive base or bulk material could then be made of a non-catalytic material, such as platinum, gold, or stainless steel.

In an embodiment, the reference electrode 110 has a larger surface area than the working electrode 120 as indicated in Fig. 1. In a particular embodiment, a relationship or ratio of the surface area of the working electrode 120 and the surface area of the reference electrode 110 is within an interval of from 1:1.1 to 1:100, preferably within an interval of from 1:1.5 to 1:10, more preferably within an interval of from 1:2 to 1:5, such as equal to 1:2.5.

By having a surface area of the reference electrode 110 that is larger than the surface area of the working electrode 120 the potential change from a floating potential is minimized during excitation. Thus, if the surface area of the reference electrodes 110 is comparatively small, the voltage drop over the electrochemical interface at the reference electrode 110 will be large. This in turn implies that the voltage drop at the working electrode 120 will be reduced with the same voltage. In order to have a working electrode 120 that is sensitive for oxygen, the reduction potential at the working electrode 120 is preferably in the interval of 0.6 to 1.4 V. Having a larger surface area of the reference electrode 110 as compared to the surface area of the working electrode 120 means that the voltage drop at the reference electrode 110 can be assumed to be at least close to zero. If the surfaces areas of the working electrode 120 and reference electrode 110 instead are, for instance, the same the measurement voltage has to be increased beyond the preferred interval. However, in such a case, the voltage drop at the reference electrode 110 will not be stable and the cross-sensitivity for, among others, temperature and salts in the blood as well as oxidation reactions will be more dominant in the measured net charge. Hence, the accuracy in the determination of pO₂ is increased by having a larger surface area of the reference electrode 110.

The larger surface area of the reference electrode 110 as compared to the surface area of the working electrode 120 could be achieved by having larger dimensions of the reference electrode 110 as compared to the working electrode 120, such as diameter, length, height, etc. depending on the shapes of the reference electrode 110 and working electrode 120.

In an embodiment, the working electrode 120 is a ring electrode 120 as shown in Fig. 1, preferably a gold ring electrode 120. In this embodiment, the reference electrode 110 is a ring electrode 110, preferably an Ag/AgCl ring electrode 110. In such an embodiment, the larger surface area of the reference ring electrode 110 can be achieved by having a larger height or length of the reference ring electrode 110 as compared to the working ring electrode 120. The two ring electrodes 110, 120 could be in the form of two cylinders having a same diameter and provided in connection with a distal end 101 of an intravascular catheter 100. The reference ring electrode 110 thereby has a larger height of its cylinder as compared to the working ring electrode 120 to thereby present a larger Ag/AgCl surface area as compared to the gold surface area of the working ring electrode 120.

An intravascular pO₂ sensor 10 that could be used according to the embodiments is disclosed in U.S. Patent No. 11,099,151, the teaching of which with regard to such a sensor is incorporated herein by reference. In particular, the intravascular pO₂ sensor 10 comprises a working electrode 120 configured to be in contact with blood and a reference electrode 110 configured to be in contact with blood and having i) a surface area that is larger than a surface area of the working electrode 120, and ii) a surface made of Ag/AgCl. The intravascular pO₂ sensor 10 also comprises a retaining circuitry configured to temporarily retain a floating voltage between the working electrode 120 and the reference electrode 110 and a measurement voltage circuitry comprising a voltage source configured to apply a measurement voltage between the working electrode 120 and the reference electrode 110 during a first measurement period causing dissolved oxygen in the blood to react by reduction at a surface of the working electrode 120 to produce an evoked current into the working electrode 120. The intravascular pO₂ sensor 10 further comprises a floating voltage circuitry configured to apply a voltage between the working electrode 120 and the reference electrode 110 equal to the temporarily retained floating voltage during a second measurement period immediately following and of an equal duration as the first measurement period to produce a current out from the working electrode 120. The intravascular pO₂ sensor 10 also comprises a generating circuitry configured to generate a signal representative of intravascular pO₂ in the blood based on a measured net charge to the working electrode 120 equal to a sum of a charge transferred to the working electrode 120 during at least a last part of the first measurement period and a charge transferred to the working electrode 120 during at least a last part of the second measurement period.

The temperature sensor 40 could be any temperature sensor that is configured to measure the temperature of the subject. Various such temperature sensors 40 are known in the art and could be used according to the invention including, but not, limited to a thermocouple, a thermistor, a temperature probe and an optical temperature sensor.

A thermocouple, also known as a thermoelectrical thermometer, is an electrical device comprising two dissimilar electrical conductors forming an electrical junction. A thermocouple produces a temperaturedependent voltage as a result of the Seebeck effect, and this voltage can be interpreted as a measure of temperature.

A thermistor is a type of resistor whose resistance is strongly dependent on temperature. Thermistors are generally divided based on their conduction model. Negative Temperature Coefficient (NTC) thermistors have less resistance at higher temperatures, while Positive Temperature Coefficient (PTC) thermistors have more resistance at higher temperatures. In an embodiment, the thermistor is an NTC thermistor.

A temperature sensor 40 in the form of a thermocouple or thermistor, in particular thermocouple, can be used to measure body temperature of the subject. For instance, the thermocouple or thermistor 130 could be arranged in an intravascular catheter 100 configured to be inserted into a blood vessel of the subject, such as integrated into the intravascular catheter 100 as shown in Fig. 1. The thermocouple or thermistor 130 does not need to be in direct contact with the blood. In clear contrast, it is exposed, by being integrated into the intravascular catheter 100, to the heating of the blood flowing past the thermocouple or thermistor 130 in the intravascular catheter 100. A very accurate core body temperature is generated by such a sensor integration.

The temperature sensor 40 does not necessarily have to be invasively arranged on an intravascular catheter 100. A temperature sensor 40 in the form of a temperature probe attached to or positioned on the subject's skin could alternatively be used to derive a temperature of the subject. Also non-contact temperature sensors 40 in the form of optical sensors could be used. As an example, an infrared (IR) temperature sensor enables accurate non-contact temperature measurement. The sensing element of such an IR temperature sensor is often composed of multiple thermocouples on a chip to measure a subject's infrared energy. Also other types of optical temperature sensors, such as optical fiber temperature sensors could be used. Such optical fiber temperature sensors are often based on fiber Bragg gratings and the operation principle is that the temperature affects the Bragg wavelength, i.e., the wavelength of peak reflectivity.

Fig. 1 illustrates an intravascular catheter 100 comprising an electrochemical pO₂ sensor realized by two ring electrodes 110, 120 integrated on the catheter tube 102 and a temperature sensor in the form of a thermocouple 130 integrated into a catheter lumen 103 according to an embodiment. The ring electrodes 110, 120 and thermocouple 130 are arranged in connection with a distal end 101 of the intravascular catheter 100 and are connected to electronic units through thin connecting wires 115, 125, 135 that run through the catheter lumen 103 to an electric contact 150 at a proximal end 105 of the intravascular catheter 100. In an embodiment, the intravascular catheter 100 comprises a fluid lumen 104 and a wire lumen 103. In such an embodiment, the wire lumen 103 comprises the connecting wires 115, 125, 135 and is closed at the distal end 101 of the intravascular catheter 10. Hence, blood will not access the wire lumen 103 when inserted into a blood vessel of the subject. A fluid lumen 104 is preferably included in the intravascular catheter 100 for blood sampling. The fluid lumen 104 could also be used to insert a guide wire into the intravascular catheter 100 through a luer contact 140 at the proximal end 105 of the intravascular catheter 100. The guide wire facilitates insertion/extrusion of the intravascular catheter 100 into/out from a blood vessel of the subject. This fluid lumen 104 may optionally be opened, such as in connection with the distal end 101 of the intravascular catheter 100.

The intravascular catheter 100 of Fig. 1 thereby houses components of the intravascular pO₂ sensor 10 and the temperature sensor 40 and can thereby be used to obtain measurements of pO₂ and temperature of a subject.

The SO₂ could be measured using various SO₂ sensors 20. There are both non-invasive SO₂ sensors and invasive SO₂ sensors that could be used according to the embodiments. An example of the former is a pulse oximeter. Pulse oximetry is a non-invasive method for monitoring a subject's oxygen saturation. The most common approach is transmissive pulse oximetry. In this approach, a sensor device is placed on a thin part of the subject's body, usually a fingertip or earlobe, or an infant's foot. The sensor device comprises a light source that transmits two wavelengths of light through the body part to a photodetector of the sensor device. The photodetector measures the changing absorbance at each of the wavelengths, allowing it to determine the absorbances due to the pulsing arterial blood alone. Another form of pulse oximetry is reflectance pulse oximetry. The sensor device comprises a light source configured to emit infrared and red light, which passes into a tissue of the subject and is reflected by the underlying bone. The reflected light is detected by a photodetector. Pulse oximeters measure so-called peripheral oxygen saturation (SpO₂) as a representation of the blood oxygen saturation, such as arterial oxygen saturation (SaO₂). Peripheral oxygen saturation as measured by pulse oximeters are often regarded sufficiently correlated with blood oxygen saturation to thereby be used as a representation of blood oxygen saturation.

In an embodiment, the SO₂ sensor 20 is an intravascular SO₂ sensor 20. In such an embodiment, the intravascular SO₂ sensor 20 is configured to measure blood oxygen saturation, such as SaO₂ or venous oxygen saturation (SvO₂), preferably SaO₂.

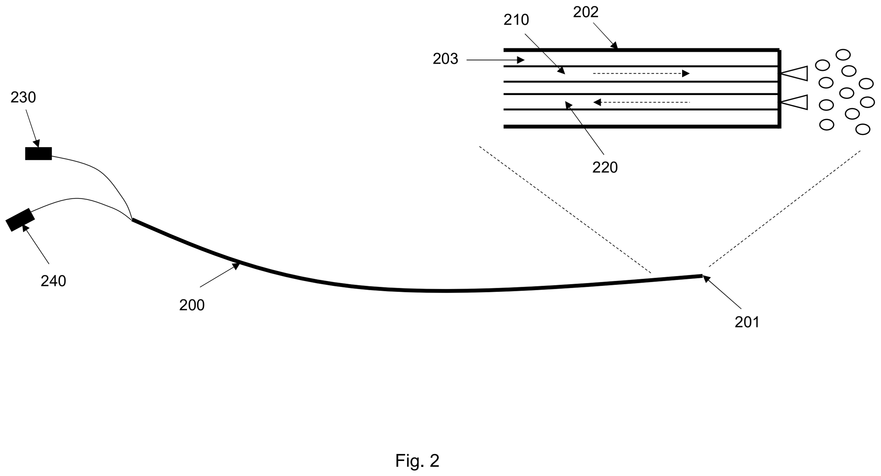

In an embodiment, the intravascular SO₂ sensor 20 comprises, see Fig. 2, a light source 230 and an efferent optical fiber 210 in optical connection with the light source 230 and configured to transmit light from the light source 230 into the blood of the subject. The intravascular SO₂ sensor 20 also comprises a light detector 240 and an afferent optical fiber 220 in optical connection with the light detector 220 and configured to transmit reflected light from the blood of the subject to the light detector 220.

The two optical fibers 210, 220 could be arranged in a lumen 203 of the catheter tubing 202 of an intravascular catheter 200 as shown in Fig. 2. The two optical fibers 210, 220 preferably end at or in connection with the distal end 201 of the intravascular catheter 200 and then run along the length of the intravascular catheter 200, or at least a portion thereof, to thereby be connected to the light source 230 and the light detector 240, respectively.

Optical connection as used herein means that light from the light source 230 is transmitted through the efferent optical fiber 210 connected to the light source 230 and that light reflected from the blood of the subject is transmitted through the afferent optical fiber 220 to the connected light detector 240.

The light source 230 of the intravascular SO₂ sensor 20 could comprise multiple, i.e., at least two, monochromatic light sources or a light source configured to generate light within a defined wavelength interval. Thus, the light source 230 can generate multiple monochromatic wavelengths or span a specific wavelength interval where the change in color properties of blood is most evident due to the amount of oxygen absorbed by the blood. For instance, the intravascular SO₂ sensor 20 could comprise two parallel optical fibers 210, 220 in an intravascular catheter 200. One fiber 210 is connected to a light source 230 that can be composed of multiple monochromatic light sources or one light source that covers a frequency interval in the visible region 400 nm to 700 nm and optionally further up to the infrared region 900 nm. The other optical fiber 220 is connected to a light detector 240, such as in the form of a photo diode or photo spectrometer, which measures the amplitude of the reflected light in each wavelength of the light source 230. Absorption or reflection of light in this frequency region responds to the color changes in blood due to different levels of oxygenation. Absorption or reflection of at least two wavelengths is preferably used to estimate SO₂. For instance, the wavelengths 665 nm and 695 nm could be used to measure blood SO₂.

Hence, in an embodiment, the multiple monochromatic light sources comprise a 665 nm light source and a 695 nm light source. In another embodiment, the light source is configured to generate light within defined wavelength interval encompassing 665 nm and 695 nm.

Such an optical fiber implementation of the SO₂ sensor 20 is advantageous since it is in direct contact with blood and, thus, very accurate, and can in contrast to a non-invasive pulse oximeter, be used to measure arterial or venous SO₂.

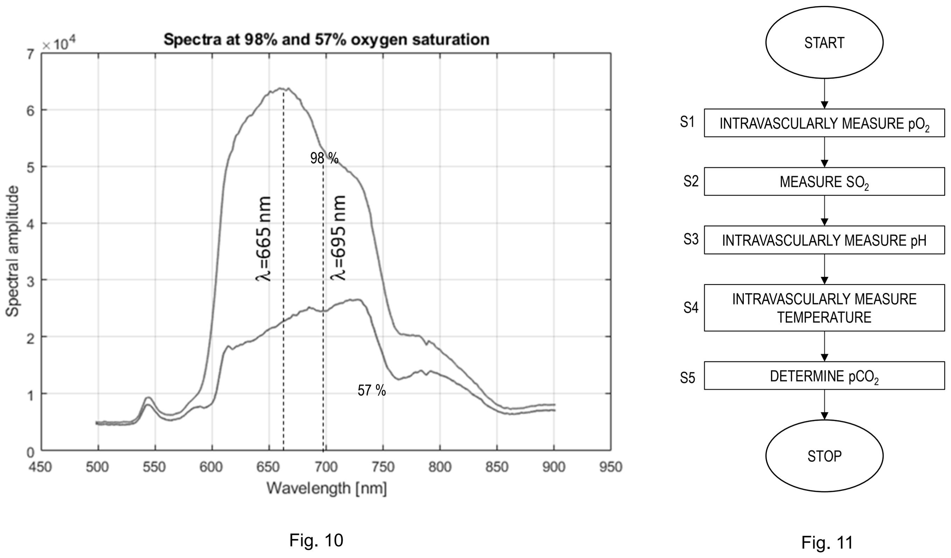

To derive the SO₂ value, the intravascular SO₂ sensor 20 uses reflected light from two different wavelengths in the red and infrared region (λ₁=665 nm and k₂=695 nm). Two amplitudes (A₁ amplitude at λ₁ and A2 amplitude at λ₂) are retrieved where the optical change is most significant when the oxygen saturation changes. The SO₂ value can then be derived as SO₂ = kX + B, wherein X = (A₁ - A₂)/A₂, k is a calibration constant and B represents an offset value corresponding to X at SO₂ = 0. Fig. 10 illustrates the spectrum at different oxygen saturations (98% and 57 % oxygen saturation) showing large variation in the wavelength 500 nm to 900 nm. Hence, as shown in Fig. 10, any two wavelengths within the interval of from 500 nm up to 900 nm, preferably within 600 nm up to 750 nm could be used by the intravascular SO₂ sensor 20. Hence, the embodiments are not limited to usage of the wavelengths 665 nm and 695 nm for the light source 230.

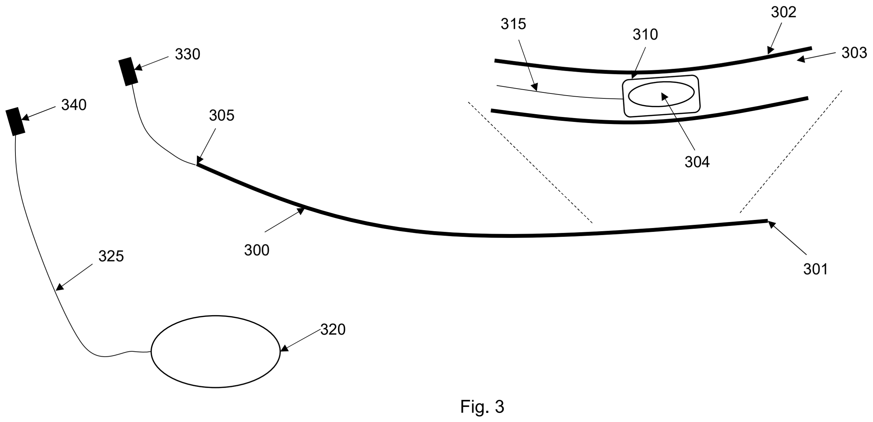

In an embodiment, the intravascular pH sensor 30 is an intravascular electrochemical sensor 30 comprising, see Fig. 3, a working electrode 310 configured to be contact with the blood of the subject and a reference electrode 320 configured to be in contact with the subject.

The working electrode 310 of the intravascular pH sensor 30 is, thus, configured to be in contact directly with the circulating blood of the subject. The reference electrode 320 is configured to be in contact with the patient, such as in the circulating blood, or, as indicated in Fig. 3, on the skin of the subject.

The working electrode 310 preferably comprises a pH sensor that could be realized as an ion-sensitive field-effect transistor (ISFET), preferably a non-glass ISFET transistor. In such an embodiment, for any change of hydrogen ions in the circulating blood, the potential of the ISFET transistor will change and, thus, indicate a change in pH value of the blood. The reference electrode 320 is stable and should be in contact with the subject and can be realized as an invasive electrode fitted to an intravascular catheter or as a patch electrode 320 attached to the skin of the subject.

Fig. 3 illustrates an intravascular catheter 300 for invasive measurement of pH value in blood according to an embodiment. The pH sensor 30 comprises an ISFET transistor 310 positioned in a catheter lumen 303 of the catheter tube 302 where an opening 304 in the catheter tube 302 enables the pH sensor to be in direct contact with blood. A reference electrode 320 is in contact with the subject and can be in the form of a patch electrode 320 attached to the skin or an electrode on the intravascular catheter 310. The ISFET transistor 310 is electrically connected to an electric contact 330 at the proximal end 305 of the intravascular catheter 300 by an electrical wire 315 running in the catheter lumen 303. Correspondingly, the patch electrode 320 is electrically connected to an electric contact 340 by an electrical cable 325.

As discussed in the foregoing, the relationship between oxygen tension and oxygen saturation in human blood depends on several blood properties including, pCO₂, pH and temperature. The actual value of pCO₂ can be determined at any values of temperature, pH, and pO₂ when the value of pO₂ at nominal conditions (temperature = 37°C, pH = 7.4 and pCO₂ = 5.3 kPa) is known. The equation to determine the pCO2 is: ${pCO}_{2} = {pCO}_{2}^{ref} \times {\left(\frac{{pO}_{2}^{actual}}{{pO}_{2}^{nom}}\right)}^{a 1} \times exp \left(a2⋅\left({pH}^{ref}-pH\right)+a3⋅\left(T-{T}^{ref}\right)\right)$ wherein exp is the exponential function and, a1, a2, and a3 are positive decimal numbers.

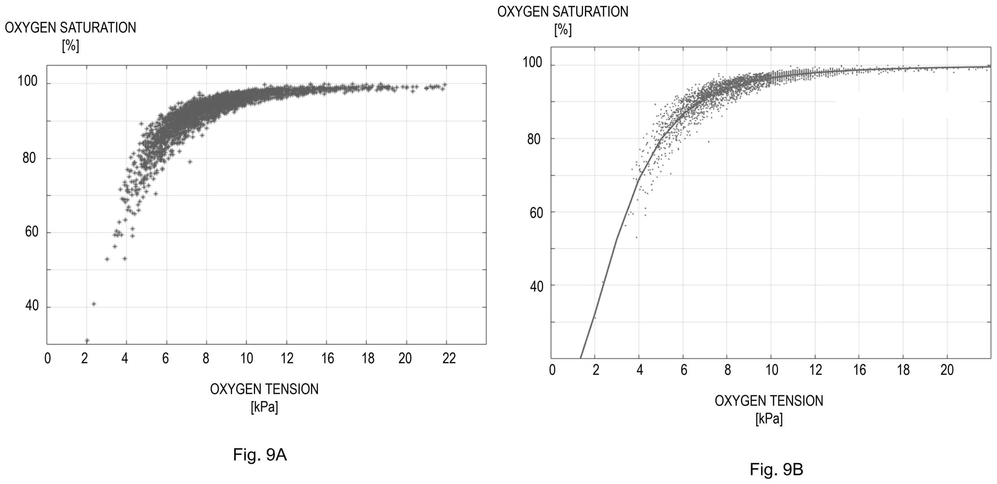

In this expression [1], ${pO}_{2}^{actual}$ represents the intravascular oxygen tension as measured by the intravascular pO₂ sensor 10, *T* is the temperature measured by the temperature sensor 40 and *pH* is the pH measured in blood by the intravascular pH sensor 30. ${pCO}_{2}^{ref}$ represents nominal CO₂, i.e., 5.3 kPa, *pH^{ref}* represents nominal pH, i.e., 7.4 and *T^{ref}* represents nominal T, i.e., 37°C. The nominal oxygen tension ${pO}_{2}^{nom}$ can be obtained from a mathematical expression of the nominal oxygen saturation curve as derived from a dataset of measurements obtained from healthy volunteers with the temperature equal to about 37°C, blood pH about 7.4 (7.35-7.45) and pCO₂ about 5.3 kPa (4.5-6.0 kPa). Fig. 9A schematically illustrates the distribution of pO₂ and SO₂ values as obtained from such healthy volunteers. Fig. 9B illustrates a sigmoid curve fitted to the distribution of pO₂ and SO₂ values. This sigmoid curve can be represented by the mathematical expression, ${SO}_{2} = 100 \times \left(\frac{{pO}_{2}^{3} + {αpO}_{2}}{{pO}_{2}^{3} + {βpO}_{2} + γ}\right)$

The parameters α, β and γ in [2] can the determined by the best fit of the sigmoid curve to the dataset as shown in Fig. 9B giving α = 5.0, β = 5.0 and γ = 40.

The nominal oxygen tension ${pO}_{2}^{nom}$ to be used in expression [1] to determine the intravascular pCO₂ can then be calculated from the expression [2] using the measured SO₂ or from a look-up table generated from expression [2] and mapping nominal oxygen tension values for various oxygen saturation values.

The present invention is not limited to the usage of the expressions [1] and [2] above. Correspondingly, other mathematical expressions representing the sigmoid curve than expression [2] could be used according to the embodiments. Furthermore, other values of the parameters α, β and γ could be determined based on a dataset similar to the one described above. For instance, expression [1] can be represented as linear functions where the non-linear terms in each expression have been linearized in the proximity of one or several working points. By applying standard mathematical linearization to one or both expressions, equivalent results can be obtained by applying the linearized expressions in several working points represented in a so-called gain schedule.

The nominal ODC described above and represented by the expression [2] is a general representation. The affinity of hemoglobin may vary between different subjects and the ODC is preferably individually calibrated to retrieve accurate pCO₂ values using the expression [1]. To derive an individual ODC, the nominal pO₂ value is broken out from the expression [1], ${pO}_{2}^{nom} = {pO}_{2}^{actual} \times {\left(\frac{{pCO}_{2}^{ref}}{{pCO}_{2}}\right)}^{k 3} \times \left({exp}^{k 1 \left(pH-{pH}^{ref}\right) + k 2 \left({T}^{ref}-T\right)}\right)$

The nominal pO₂ value is normally in the range 5 to 15 kPa. The calibration values above are preferably retrieved by a reference system, such as a BGA. The BGA measures accurate calibration values from a blood sample extracted from the subject. The temperature cannot be retrieved from the BGA but rather sampled directly from the temperature sensor 40. When accurate calibration values have been obtained, the nominal pO₂ can be derived from expression [3]. The nominal pO₂ value is the pO₂ value that is expected in a nominal ODC curve that is not exposed to a right shift or left shift i.e., when properties of the blood are in normal conditions described by the reference values.

When an accurate estimate of the nominal pO₂ has been obtained, the calibration parameter γ in expression [2] can be derived by inserting the accurate value of SO₂ that corresponds to the pO₂ calibration value. The SO₂ is preferably obtained from the BGA and measured in the same blood sample as the other calibration parameters. Alternatively, the SO₂ value can be obtained directly from the system 1. The SO₂ value is preferably sampled at the same moment as the blood sample is taken to correspond accurately to the other calibration parameters obtained from the BGA.

The parameter γ is derived by modifying expression [2] as follows, $γ = \left(\frac{100}{{SO}_{2}}-1\right) {pO}_{2}^{3} + \left(\frac{100 α}{{SO}_{2}}-β\right) {pO}_{2}$

The best fit values of α and β according to above can then be inserted into the expression [4] to derive the calibrated value of γ. This calibrated value of γ can then be used to derive the nominal pO₂ for any measured SO₂ value and the intravascular pCO₂ can be determined from expression [1].

Hence, there is generally an individual, i.e., subject-specific, variability of affinity in blood and thereby an individual ODC. Accordingly, calibration parameters are preferably used by the system 1 in the determination of pCO₂ to compensate for such individual ODC characteristics.

In such an embodiment, the memory 60 of the system 1 comprises at least one calibration parameter calculated by the processor 50 based on measured pO₂, SO₂, pH and temperature and a pCO₂ estimate measured in a blood sample from the subject using a blood gas analyzer. The memory 50 also comprises instructions executable by the processor 50 to cause the processor 50 to determine the intravascular pCO₂ for the subject based on the measured pO₂, SO₂, pH, temperature and the at least one calibration parameter.

Hence, in such an embodiment, the processor 50 is configured to determine the intravascular pCO₂ as a function f'( ) of the measured pO₂, SO₂, pH and temperature parameters and the at least one calibration parameters, pCO2 = f'(pO2, SO2, pH-value, temperature, calibration parameter(s)).

In an embodiment, the memory 60 comprises instructions executable by the processor 50 to cause the processor 50 to determine a nominal pO₂ based on the measured SO₂ and determine the intravascular pCO₂ for the subject based on the determined nominal pO₂ and the measured pO₂, pH and temperature.

In a particular embodiment, the memory 60 comprises instructions executable by the processor 50 to cause the processor 50 to determine a subject-specific calibration parameter based on the measured SO₂ and pO₂ and determine the nominal pO₂ based on the measured SO₂ and the subject-specific calibration parameter.

In an embodiment, the memory 60 comprises instructions executable by the processor 50 to cause the processor 50 to determine intravascular pCO₂ for the subject using equation [1].

In an embodiment, the system 1 comprises a display screen 80 as shown in Fig. 4. This display screen 80 is wirelessly connected or connected by wire to the processor 50. The memory 60 then comprises instructions executable by the processor 50 to cause the processor 50 to display the determined intravascular pCO₂ on the display screen 80.

The display screen 80 could then display pCO₂ values as determined by the processor 50. Alternatively, or in addition, the display screen 80 could display a trend curve of determined pCO₂ over time. The display screen 80 may optionally also display values and/or trend curves of the other parameters measured by the system 1, i.e., pO₂, SO₂, temperature, and/or pH-value.

In an embodiment, the processor 50 could be configured to first verify whether the determined pCO₂ is within acceptable realistic values before displaying the pCO₂ value on the display screen 80. In such an embodiment, the memory 60 comprises instructions executable by the processor 50 to cause the processor 50 to determine whether the determined intravascular pCO₂ is within a predefined interval and display the determined intravascular pCO₂ on the display screen 80 if the determined intravascular pCO₂ is within the predefined interval and not display the determined intravascular pCO₂ on the display screen 80 if the determined intravascular pCO₂ is outside of the predefined interval. An illustrative, but non-limiting, example of such a predefined interval could be from 1 kPa up to 25 kPa, preferably from 3 kPa up to 15 kPa.

Fig. 4 illustrates a processing device, such as computer 90, comprising the processor 50 and the memory 60. The computer 90 optionally, but preferably, comprises an input and output (I/O) unit 70 for conducting communication with external devices, such as the sensors 10, 20, 30, 40 and the display screen 80. The I/O unit 70 could be configured to wireless communicate with the external devices and may be implemented in the form of a transmitter and a receiver, or a transceiver. Alternatively, the I/O unit 70 could be in the form of an input and output port for wired connection to external devices. It is also possible to have an I/O unit 70 that comprises at least one transmitter and receiver, or transceiver, for wireless communication with at least one external device and at least one I/O port for wired connection with at least one other external device. Hence, the I/O unit 70 could be any communications interface configured for communication with other entities, functions, nodes, devices, and modules, such as the sensors 10, 20, 30, 40 and the display screen 80.

The processor 50 is provided using any combination of one or more of a suitable central processing unit (CPU), multiprocessor, microcontroller, digital signal processor (DSP), etc., capable of executing software instructions stored in a computer program product, e.g., in the form of a storage medium or memory 60. The processor 50 may further be provided as at least one application specific integrated circuit (ASIC), or field programmable gate array (FPGA).

Particularly, the processor 50 is configured to cause the system 1 to perform a set of operations, or steps, as disclosed herein. For example, the memory 60 may store the set of operations, and the processor 50 may be configured to retrieve the set of operations from the memory 60 to cause the system 1, and in particular the processor 50, to perform the set of operations. The set of operations may be provided as a set of executable instructions. Thus, the processor 50 is thereby arranged to execute operations as disclosed herein. The memory 60 may comprise persistent storage, which, for example, can be any single one or combination of magnetic memory, optical memory, solid state memory or even remotely mounted memory. The processor 50 controls the general operation of the system 1, e.g., by sending data and control signals to the I/O unit 70 and the memory 60, by receiving data from the I/O unit 70, and by retrieving data and instructions from the memory 60.

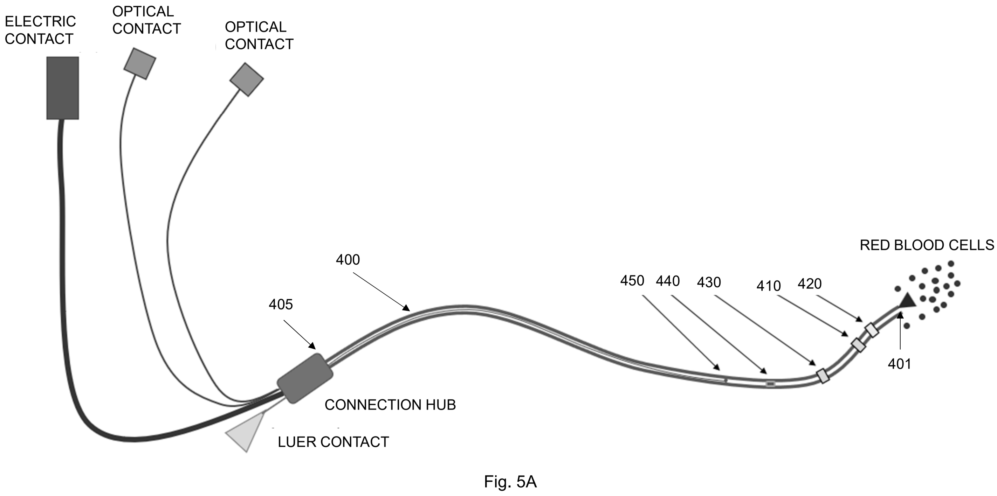

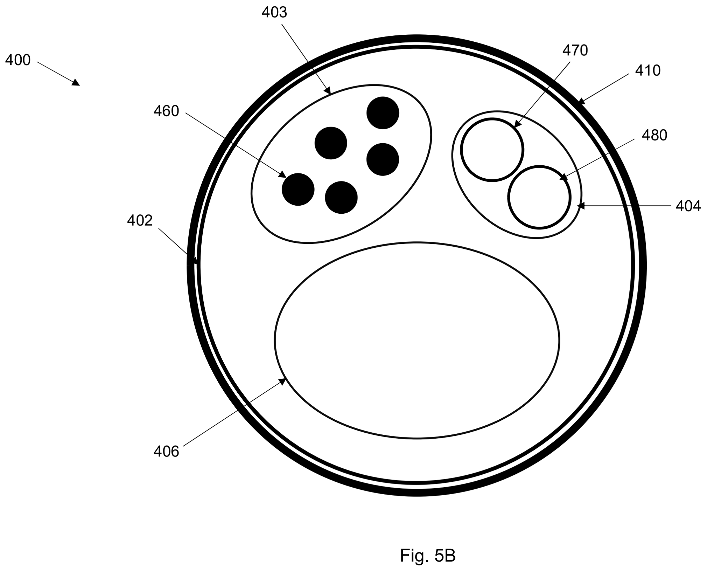

In an embodiment, the sensors 10, 20, 30, 40 of the system 1 are integrated into a medical device, such as an intravascular catheter 400, see Figs. 5A and 5B. In such an embodiment, the intravascular catheter 400 comprises a working electrode 420 and a reference electrode 410 for measurement of pO₂, an ISFET pH sensor 440 and optional reference electrode 430 for the ISFET pH sensor 440 for pH measurements, optical fibers 470, 480 for measurement of SO₂, and a thermocouple or thermistor 450 for measurement of temperature. The temperature sensor 450 can be integrated in the plastic material, but the other sensors elements 410, 420, 430, 440, 470, 480 are preferably in direct contact with the circulating blood.

The pO₂ sensor is, in an embodiment, realized as two ring electrodes 410, 420 mounted at the distal end 401 of the catheter tube 402. The working electrode 420 is preferably made of pure gold and the reference electrode 410 is made of Ag/AgCl. Both electrodes 410, 420 are connected to an electric contact at the proximal end 405 of the intravascular catheter 400 by two thin electrical wires 460 that run through a dedicated wire lumen 403 in the catheter tube 402. A pO₂ electronic module is connected to the electric contact to enable pO₂ measurement.

The pH sensor is, in an embodiment, realized as an ISFET transistor 440 and a reference electrode 430. The ISFET transistor 440 is inserted into a catheter lumen where a small opening "window" exposes the ISFET transistor 440 directly to blood surrounding the intravascular catheter 400. The reference electrode 430 is, in an embodiment, a ring electrode mounted to the catheter tube 402.

The SO₂ sensor is, in an embodiment, realized by two parallel optical fibers 470, 480 that run through a catheter lumen 404 in the catheter tube 402 and exit the catheter tube 402 at the distal tip where the optical fibers 470, 480 are exposed to the surrounding blood. The optical fibers 470, 480 preferably run through a dedicated lumen 404. The optical fibers 470, 480 are at the proximal end 405 of the intravascular catheter 400 connected to a light source and a photo spectrometer or photo diode through optical contacts.

The temperature sensor is, in an embodiment, realized by a thermocouple 450 embedded into a closed catheter lumen 403. The thermocouple 450 is preferably completely integrated in the plastic tube 402 and is not in contact with the subject's blood. The catheter tube 402 will warm up to the body temperature when inserted into the subject's blood vessel and the thermocouple 450 will measure the temperature of the catheter tube 402, thus an indirect but very accurate measurement of body temperature. The thermocouple 450 is connected to an electric contact at the proximal end 405 of the intravascular catheter 400 and further to electronics to measure and derive the actual temperature.

Hence, in an embodiment, the system 1 comprises an intravascular catheter 400 comprising a working ring electrode 420 of the intravascular pO₂ sensor 10, a reference ring electrode 410 of the intravascular pO₂ sensor 10, a thermocouple or thermistor 450 of the intravascular temperature sensor 40, an efferent optical fiber 470 of the SO₂ sensor 20, an afferent optical fiber 480 of the sO₂ sensor 20 and an ISFET pH sensor 440. The intravascular catheter 400 also comprises a wire lumen 403 comprising electrical wirings 460 for the working ring electrode 420, the reference ring electrode 410, the thermocouple or thermistor 450 and the ISFET pH sensor 440 and a fiber lumen 404 comprising the efferent optical fiber 470 and the afferent optical fiber 430.

In an embodiment, the intravascular catheter 400 also comprises a reference ring electrode 430 for the ISFET pH sensor 440. In such an embodiment, the wire lumen 403 comprises electrical wiring 460 for the reference ring electrode 430.

In an embodiment, the intravascular catheter 400 comprises a flow lumen 406 configured to be in fluid contact with blood of the subject when the intravascular catheter 400 is present in a blood vessel of the subject. In an embodiment, the ISFET pH sensor 440 is in fluid contact with the flow lumen 406.

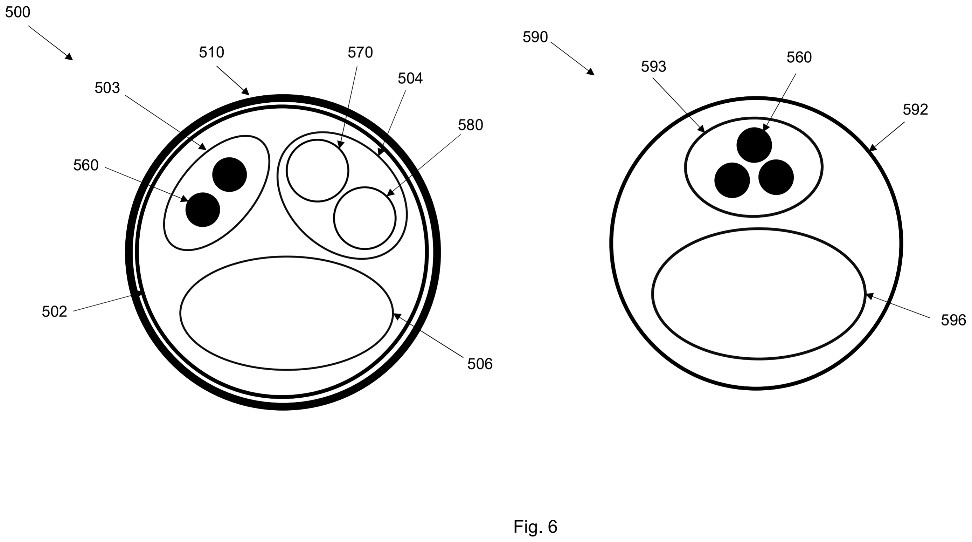

In the embodiments described above, the sensors, or portions thereof, are integrated into the same intravascular catheter 400. This intravascular catheter 400 is configured for insertion into a blood vessel of a subject, preferably an artery. In some clinical cases, thin catheter tubes need to be used in order to be inserted into subjects with small blood vessels, such as for neonatal or pediatric subjects. In such a case, the sensors, or portions thereof, can be separated between two intravascular catheters 500, 590, see Fig. 6. In such an embodiment, one of the intravascular catheters 500, 590 could be an arterial catheter, such as an umbilical arterial catheter 500, and the other intravascular catheter 590 is a venous catheter, such as an umbilical venous catheter 590.

In such an embodiment, the system 1 comprises a first intravascular catheter 500 comprising a working ring electrode of the intravascular pO₂ sensor 10, a reference ring electrode 510 of the intravascular pO₂ sensor 10 and an efferent optical fiber 570 and an afferent optical fiber 480 of the SO₂ sensor 20. The first intravascular catheter 500 also comprises a wire lumen 503 comprising electrical wirings 560 for the working ring electrode and the reference ring electrode 510 and a fiber lumen 504 comprising the efferent optical fiber 570 and the afferent optical fiber 580. The system 1 also comprises a second intravascular catheter 590 comprising an ISFET pH sensor and a thermocouple or thermistor of the intravascular temperature sensor 40. The second intravascular catheter 590 also comprises a wire lumen 593 comprising electrical wirings 560 for the thermocouple or thermistor and the ISFET pH sensor.

In an embodiment, the second intravascular catheter 590 also comprises a reference ring electrode for the ISFET pH sensor. In such an embodiment, the wire lumen 590 comprises electrical wiring 460 for the reference ring electrode.

The first and second intravascular catheters 500, 590 may optionally comprise a respective a flow lumen 506, 596 configured to be in fluid contact with blood of the subject when the first and second intravascular catheters 500, 590 are present in a blood vessel of the subject.

Fig. 11 is a flow chart illustrating a method for determining intravascular pCO₂. The method comprises intravascularly measuring pO₂ in a subject in step S1, measuring SO₂ in the subject in step S2, intravascularly measuring pH in blood of the subject in step S3 and measuring temperature of the subject in step S4. These steps S1 to S4 could be performed serially in any order or, preferably at least partly in parallel. The method also comprises determining intravascular pCO₂ for the subject based on the measured pO₂, SO₂, pH and temperature in step S5.

In an embodiment, the method also comprises determining a nominal pO₂ based on the measured SO₂. In such an embodiment, step S5 comprises determining the intravascular pCO₂ for the subject based on the determined nominal pO₂ and the measured pO₂, pH and temperature.

In an embodiment, the method further comprises determining a subject-specific calibration parameter based on the measured SO₂ and pO₂. In such an embodiment, determining the nominal pO₂ comprises determining the nominal pO₂ based on the measured SO₂ and the subject-specific calibration parameter.

In an embodiment, step S5 comprises determining the intravascular pCO₂ for the subject based on ${pCO}_{2} = {pCO}_{2}^{ref} \times {\left(\frac{{pO}_{2}^{actual}}{{pO}_{2}^{nom}}\right)}^{a 1} \times exp \left(a2⋅\left({pH}^{ref}-pH\right)+a3⋅\left(T-{T}^{ref}\right)\right) ,$ wherein
exp is the exponential function;
*pH^{ref}* represents nominal blood pH, preferably 7.4;
*T^{ref}* represents nominal blood temperature, preferably 37°C;
${pCO}_{2}^{ref}$ represents nominal blood pCO₂, preferably 5.3 kPa;
*pH* represents pH in blood of the subject measured by the intravascular pH sensor;
*T* represents temperature of the subject measured by the temperature sensor;
${pO}_{2}^{nom}$ represents the nominal pO₂ at nominal conditions with *pH^{ref}, T^{ref},* ${pCO}_{2}^{ref}$;
${pO}_{2}^{actual}$ represents pO₂ in the subject measured by the intravascular pO₂ sensor; and
a1, a2, and a3 are positive calibration parameters represented as decimal numbers.

The method as shown in Fig. 11 is preferably performed by system 1 as disclosed herein. Accordingly, the various embodiments of the system 1 described in the foregoing also applies to the method.

The system 1 has been evaluated in a patient simulator in a lab environment. Measurements have been acquired by inserting sensors 10, 20, 30, 40 above in the simulator. Human blood was circulated in the patient simulator. Right shift and left shift of the ODC was well predicted by changes in pH value, pCO₂, and temperature. Changes in oxygenation did not affect the ODC since changes in pO₂ and SO₂ followed the characteristics of the ODC determined by the used test conditions.

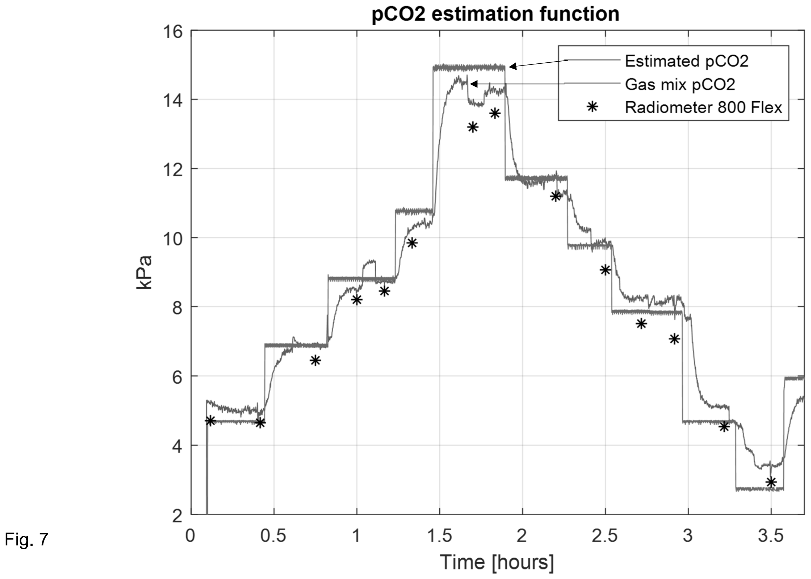

Fig. 7 is a graph showing pCO₂ estimated with the system 1 in relation to pCO₂ measured in blood samples from an ECMO system and analyzed by a BGA, and a continuous derivation of the pCO2 in the gas mix that was flushed through the oxygenator. After an initial calibration, the pCO₂ was increased in steps from 5 kPa to 15 kPa and thereafter decreased to 2 kPa and finally increased again to 6 kPa. The change was followed by a corresponding change of SO₂ and pH-value governed by the changes in affinity of the red blood cells. It took a few minutes for the new blood gas values to equilibrate in the blood and the estimated pCO₂ followed the actual value as derived by the gas mix and measured by the BGA very well.

### EXAMPLES

### EXAMPLE 1

A preterm baby arrives at the neonatal intensive unit. The neonatal physicians insert an arterial umbilical catheter and a venous umbilical catheter, see Fig. 6. The arterial catheter is equipped with two ring electrodes to measure arterial pO₂ and two optical fibers to measure arterial SO₂. The venous catheter has an integrated temperature sensor and an ISFET transistor, which is in contact with blood through a small opening (window) in the catheter tube. Both catheters are connected to a computer with built in measurement modules for pO₂, SO₂, temperature, and pH-value. An arterial blood sample is taken and analyzed with a BGA. The measured venous pH-value was converted by the computer to represent the arterial pH-value. The value of each parameter is inserted into the computer by a user. The computer uses the inserted values of pO₂, SO₂, temperature, pH-value, and pCO₂ value to calibrate the pCO₂ estimation function. The computer starts displaying real time numeric values and trends of all parameters. The supplied oxygen (FiO₂) is optimized based on the arterial values of pO₂ and SO₂ to minimize risk for complications. Based on the pCO₂ value, it is decided if the patient will need mechanical ventilation. If the patient is intubated and connected to a mechanical ventilator, the respiration rate and tidal volume is set to maintain pCO₂ within limits that further minimize risk for patient complications. Monitoring of pO₂, SO₂, and pCO₂ are used throughout the catheters time of use.

Too high oxygenation is associated with increased risk or retinal damage (ROP) for preterm patients, and too low oxygenation is associated with increased risk for brain damage and mortality.

Elevated pCO₂ is associated with several serious conditions for preterm babies such as high blood pressure leading to cerebral hemorrhage, bronchopulmonary dysplasia (BPD), necrotizing enterocolitis (NEC), and poorer neurodevelopment.

### EXAMPLE 2

A critically ill patient arrives at the intensive care unit (ICU). The ICU physician inserts a central venous catheter with ultrasound guidance through the internal jugular vein until the tip of the catheter enters the right atrium. The physician closes the incision with a suture and fixates the catheter. The catheter is connected to the computer with built in measurement modules for pO₂, SO₂, temperature, and pH-value. A venous blood gas is taken from the fluid lumen of the catheter and analyzed with a BGA. The value of each parameter is inserted to the computer by a user. The computer uses inserted values of pO₂, SO₂, temperature, pH-value, and pCO₂ value to calibrate the pCO₂ estimation function. The computer starts displaying real time numeric values and trends of all parameters. Together with the SpO₂ value from a pulse oximeter the ICU staff has now access all important arterial and venous blood-based parameters to optimize treatment of the patient. The ventilation settings can be adjusted to minimize lung damage and maintain adequate oxygenation. Effects from several complications can be minimized by early detection of changes in gas exchange caused by a respiratory failure, infection, organ failure, or heart failure. Monitoring of pO₂, SO₂, pH-value, and pCO₂ are used throughout the dwell time of the catheters.

### EXAMPLE 3

The purpose of this Example is to derive carbon dioxide tension (pCO₂) from information of pH, temperature, oxygen tension (pO₂), and oxygen saturation (SO₂) in human blood. It is further described how well the method works for blood gas data acquired in a neonatal clinical setting.

In this Example, expression [1] will be used to predict pCO₂ from the other variables. ${pCO}_{2} = {pCO}_{2}^{ref} \times {\left(\frac{{pO}_{2}^{actual}}{{pO}_{2}^{nom}}\right)}^{a 1} \times exp \left(a2⋅\left({pH}^{ref}-pH\right)+a3⋅\left(T-{T}^{ref}\right)\right)$ wherein a1, a2, and a3 are positive decimal numbers. pCO₂ represents the tension (partial pressure) of carbon dioxide in blood, pO₂ represents the tension (partial pressure) of oxygen in blood, SO₂ represents oxygen saturation in blood, ${pO}_{2}^{nom}$ represents pO₂ at nominal conditions with pH = 7.4, T = 37°C and pCO₂ = 5.3 kPa, ${pO}_{2}^{actual}$ represents pO₂ at a condition other than the nominal condition, *pH^{ref}* represents nominal pH, i.e., 7.4, *T^{ref}* represents nominal temperature, i.e., 37°C, ${pCO}_{2}^{ref}$ represents nominal pCO₂, i.e., 5.3 kPa.

A large dataset from the neonatal intensive care unit at Karolinska University Hospital (Stockholm, Sweden) was used in the analysis. The dataset was acquired at three hospitals from 2010 to 2015 and was composed of 58,147 blood measurements of pO₂, SO₂, and pH-value. The dataset was separated into two subsets where the first subset of 41,550 measurements was used to derive parameter values in expressions [3], and the second dataset of 16,597 measurements was used to validate the derived model. Values of the model parameters were optimized using Least Square Estimate (LSE). To find optimal values for k1, k2, k3 in expression [3] and thereby for a1, a2 and a3 in expression [1], expression [1] was restricted into suitable regressors [5]: $ln \left(\frac{{pCO}_{2}}{{pCO}_{2}^{ref}}\right) = a 1 \times ln \left(\frac{{pO}_{2}^{actual}}{{pO}_{2}^{nominal}}\right) + a 2 \left({pH}^{ref}-pH\right) + a 3 \left(T-{T}^{ref}\right)$

Since temperature values were not accessible by the BGA (ABL800 Flex, Radiometer), the temperature dependency was excluded from expression [5].

The output *y* and the regressors *φ* were thereby defined as follows: ${φ}_{1} = ln \left({pO}_{2}^{actual}/{pO}_{2}^{nom}\right)$ ${φ}_{2} = \left({pH}^{ref}-pH\right)$ $y = ln \left(\frac{{pCO}_{2}}{{pCO}_{2}^{ref}}\right)$ $θ = \left[a1,a2\right]$

The LSE was applied to derive the best fit of parameters a1 and a2. When optimized values for a1 and a2 were inserted into expression [5] and applied to the validation blood gas dataset, the results presented in Table 1 were obtained.

**Table 1 - GoF parameters**

| GoF parameter | Value |
|---|---|
| RMSE | 1.03 |
| STD | 0.99 |
| MAE | 0.77 |

| | |
|---|---|
| ¹root mean square error ²standard deviation ³mean absolute error | |

### EXAMPLE 4

In this Example, the same pCO₂ estimation function as described in Example 3 was evaluated with real time data in an *in vitro* environment, where data was acquired by an aspiration syringe directly from circulating human blood.

The model validation was performed in an extracorporeal membrane oxygenation (ECMO) system with circulating human blood, where the levels of pO₂ and pCO₂ could be changed by a controllable mix of gases. The gas mix was composed of O₂, N₂, air, CO₂, and controlled by valves that could deliver a specific gas flow with high accuracy. The mixture of gases was flushed through an oxygenator and thereby transferred over to the circulating blood.

Human blood was acquired from the blood central at Karolinska hospital (Stockholm, Sweden) and inserted into the ECMO system. The pCO₂ and pO₂ were changed and blood samples were taken regularly to analyze with a blood gas analyzer (BGA)) (ABL800 Flex, Radiometer). At the end of the test, the temperature of circulating blood was varied to identify a value for parameter a3 in expression [1]. The values in Table 2 were retrieved during the test.

**Table 2 - Blood gas data and pCO₂ estimates**

| pO₂ (BGA) (kPa) | SO₂ (BGA) (kPa) | pH (BGA) | T (°C) | pCO₂ (BGA) (kPa) | pCO₂ estimate (kPa) |
|---|---|---|---|---|---|
| 10.0 | 98.0 | 7.36 | 37 | 4.7 | 5.1 |
| 10.0 | 97.3 | 7.22 | 37 | 7.2 | 7.1 |
| 10.0 | 96.0 | 7.15 | 37 | 9.2 | 8.8 |
| 10.0 | 96.8 | 7.09 | 37 | 10.7 | 9.4 |
| 10.0 | 95.3 | 7.03 | 37 | 13.1 | 11.4 |
| 10.0 | 93.9 | 6.97 | 37 | 15.2 | 13.5 |
| 10.0 | 95.1 | 7.03 | 37 | 12.0 | 11.5 |
| 10.0 | 98.2 | 7.39 | 37 | 4.7 | 4.7 |
| 10.0 | 95.9 | 7.08 | 37 | 10.1 | 10.1 |
| 8.0 | 96.6 | 7.38 | 37 | 4.7 | 4.8 |
| 6.0 | 92.7 | 7.37 | 37 | 4.7 | 4.9 |
| 4.0 | 76.7 | 7.36 | 37 | 4.7 | 5.2 |
| 15.1 | 98.7 | 7.36 | 37 | 4.7 | 5.9 |
| 7.0 | 93.5 | 7.37 | 37 | 4.7 | 5.2 |
| 7.0 | 87.7 | 7.35 | 40 | 4.7 | 5.3 |
| 7.0 | 98.3 | 7.36 | 32 | 4.7 | 5.2 |

The LSE was applied to derive the best fit of parameters a3. When optimized values for a1, a2 and a3 were inserted into expression [5], the results presented in Table 3 were obtained.

**Table 3 - GoF parameters**

| GoF parameter | Value |
|---|---|
| RMSE | 0.81 |
| STD | 0.81 |
| MAE | 0.61 |

### EXAMPLE 5

In this Example, pCO₂ was estimated by real time measurements of pH, SO₂, pO₂, and temperature. The pO₂ and temperature were measured by a catheter 100 as disclosed in Fig. 1, the SO₂ was measured by a probe with optic fibers, a white light source and a photo detector, see Fig. 2, and a commercially available ISFET pH-sensor was used to measure the pH level. The sensors were inserted into the tubing of an ECMO system in circulating blood, and measurement data was acquired and used to derive pCO₂.

Human blood was injected into the ECMO tubing system and heated up to 37°C while circulated by a peristaltic pump. A gas mix composed of air, nitrogen, and carbon dioxide was flushed through the oxygenator. A blood sample was acquired and analyzed with a BGA (ABL800 Flex, Radiometer). The BGA results showed an unphysiologically low pH level at 6.8. To neutralize the pH to a physiologic range, covering pH values of 7.2 to 7.6, a small amount of water mixed with bicarbonate was injected into the circulating blood. When the pH level was stabilized at 7.35, the gas mix was set to pO₂ = 10.0 kPa and pCO₂ = 5.0 kPa. Blood gases in the circulating blood were stabilized to the set gas mix.

To stress the pCO₂ estimation, the pO₂ and pCO₂ in the gas mix were changed in a stepwise manner. As the pCO₂ changed in the circulating blood, the pH level changed accordingly, and as the pH level changed the blood affinity was affected, which also changed the SO₂ although the pO₂ was stable.

The pCO₂ estimation was tested for changes in pO₂ and pCO₂. The estimated pCO₂ value shall not be affected by changes in pO₂ and only respond to changes in pCO₂. With the optimized parameter values in the pCO₂ expression as determined in Examples 3 and 4, the estimated pCO₂ value was only slightly changed when the pO₂ level changed but followed the changes in pCO₂ very well as seen in Fig. 7. It was thereby validated that the estimated pCO₂ fit well to reality.

The embodiments described above are to be understood as a few illustrative examples of the present invention. It will be understood by those skilled in the art that various modifications, combinations, and changes may be made to the embodiments without departing from the scope of the present invention. In particular, different part solutions in the different embodiments can be combined in other configurations, where technically possible. The scope of the present invention is, however, defined by the appended claims.

## Claims

1. A system (1) for determining intravascular partial pressure of carbon dioxide, pCO₂, the system (1) comprising:
an intravascular oxygen tension, pO₂, sensor (10) configured to measure pO₂ in a subject;
an oxygen saturation, SO₂, sensor (20) configured to measure SO₂ in the subject;
an intravascular pH sensor (30) configured to measure pH in blood of the subject;
a temperature sensor (40) configured to measure temperature of the subject;
a processor (50); and
a memory (60) coupled to the processor (50) and comprising instructions executable by the processor (50) to cause the processor (50) to determine intravascular pCO₂ for the subject based on the measured pO₂, SO₂, pH and temperature.

2. The system according to claim 1, wherein
the intravascular pO₂ sensor (10) is configured to measure pO₂ in real time in the subject and generate a real-time pO₂ estimate;
the SO₂ sensor (20) is configured to measure SO₂ in real time in the subject and generate a real-time SO₂ estimate;
the intravascular pH sensor (30) is configured to measure pH in real time in blood of the subject and generate a real-time pH estimate;
the temperature sensor (40) is configured to measure temperature in real time of the subject and generate a real-time temperature estimate;
the memory (60) comprises instructions executable by the processor (50) to cause the processor (50) to determine a real-time intravascular pCO₂ estimate based on the real-time pO₂ estimate, the real-time SO₂ estimate, the real-time pH estimate and the real-time temperature estimate.

3. The system according to claim 1 or 2, wherein the intravascular pO₂ sensor (10) is an intravascular electrochemical sensor (10) comprising a working electrode (120) and a reference electrode (110) configured to be in contact with the blood of the subject.

4. The system according to claim 3, wherein
the working electrode (120) comprises a surface made of gold, preferably wherein the working electrode (120) is a god ring electrode (120);
the reference electrode (110) comprises a surface made of Ag/AgCl, preferably wherein the reference electrode (110) is an Ag/AgCl ring electrode (110); and
the reference electrode (110) has a larger surface area than the working electrode (120).

5. The system according to any one of claims 1 to 4, wherein the SO₂ sensor (20) is an intravascular SO₂ sensor (20).

6. The system according to claim 5, wherein the intravascular SO₂ sensor comprises:
a light source (230), preferably multiple monochromatic light sources, more preferably a 665 nm light source and a 695 nm light source, or a light source configured to generate light within a defined wavelength interval, more preferably within the defined wavelength interval encompassing 665 nm and 695 nm;
an efferent optical fiber (210) in optical connection with the light source (230) and configured to transmit light from the light source (230) into the blood of the subject;
a light detector (240), preferably a photo diode or a photo spectrometer; and
an afferent optical fiber (220) in optical connection with the light detector (240) and configured to transmit reflected light from the blood of the subject to the light detector (240).

7. The system according to any one of claims 1 to 6, wherein the intravascular pH sensor (30) is an intravascular electrochemical sensor (30) comprising a working electrode (310), preferably comprising an ion-sensitive field-effect transistor, ISFET, configured to be in contact with the blood of the subject and a reference electrode (320) configured to be in contact with the subject.

8. The system according to any one of claims 1 to 7, wherein the temperature sensor (40) comprises a thermocouple or a thermistor (130), preferably arranged in a catheter (100) configured to be inserted into a blood vessel of the subject, configured to measure body temperature of the subject.

9. The system according to any one of claims 1 to 8, wherein the memory (60) comprises instructions executable by the processor (50) to cause the processor (50) to:
determine a nominal pO₂ based on the measured SO₂; and
determine the intravascular pCO₂ for the subject based on the determined nominal pO₂ and the measured pO₂, pH and temperature.

10. The system according to claim 9, wherein the memory (60) comprises instructions executable by the processor (50) to cause the processor (50) to:
determine a subject-specific calibration parameter based on the measured SO₂ and pO₂; and
determine the nominal pO₂ based on the measured SO₂ and the subject-specific calibration parameter.

11. The system according to claim 9 or 10, wherein the memory (60) comprises instructions executable by the processor (50) to cause the processor (50) to determine the intravascular pCO₂ for the subject based on ${pCO}_{2} = {pCO}_{2}^{ref} \times {\left(\frac{{pO}_{2}^{actual}}{{pO}_{2}^{nom}}\right)}^{a 1} \times exp \left(a2⋅\left({pH}^{ref}-pH\right)+a3⋅\left(T-{T}^{ref}\right)\right) ,$ wherein
exp is the exponential function;
*pH*^{*r*e*f*} represents nominal blood pH, preferably 7.4;
*T^{ref}* represents nominal blood temperature, preferably 37°C;
${pCO}_{2}^{ref}$ represents nominal blood pCO₂, preferably 5.3 kPa;
*pH* represents pH in blood of the subject measured by the intravascular pH sensor (30);
*T* represents temperature of the subject measured by the temperature sensor (40);
${pO}_{2}^{nom}$ represents the nominal pO₂ at nominal conditions with *pH^{ref}*, *T^{ref}*, ${pCO}_{2}^{ref}$;
${pO}_{2}^{actual}$ represents pO₂ in the subject measured by the intravascular pO₂ sensor (10); and
a1, a2, and a3 are positive calibration parameters represented as decimal numbers.

12. The system according to any one of claims 1 to 11, further comprising an intravascular catheter (400) comprising:
a working ring electrode (420) of the intravascular pO₂ sensor (10);
a reference ring electrode (410) of the intravascular pO₂ sensor (10);
a thermocouple or thermistor (450) of the intravascular temperature sensor (40);
an efferent optical fiber (470) of the SO₂ sensor (20);
an afferent optical fiber (480) of the SO₂ sensor (20);
an ion-sensitive field-effect transistor, ISFET, pH sensor (440);
a wire lumen (403) comprising electrical wirings (460) for the working ring electrode (420), the reference ring electrode (410), the thermocouple or thermistor (450) and the ISFET pH sensor (440); and
a fiber lumen (404) comprising the efferent optical fiber (470) and the afferent optical fiber (480).

13. The system according to claim 12, wherein
the intravascular catheter (400) comprises a reference electrode (430) for the ISFET pH sensor (440); and
the wire lumen (403) comprises electrical wiring (460) for the reference electrode (430) for the ISFET pH sensor (440).

14. The system according to any one of claims 1 to 11, further comprising:
a first intravascular catheter (500) comprising:
a working ring electrode of the intravascular pO₂ sensor (10);
a reference ring electrode (510) of the intravascular pO₂ sensor (10);
an efferent optical fiber (570) of the SO₂ sensor (20);
an afferent optical fiber (580) of the SO₂ sensor (20);
a wire lumen (503) comprising electrical wirings (560) for the working ring electrode and the reference ring electrode (510); and
a fiber lumen (504) comprising the efferent optical fiber (570) and the afferent optical fiber (580); and
a second intravascular catheter (590) comprising:
an ion-sensitive field-effect transistor, ISFET, pH sensor (30);
a thermocouple or thermistor of the intravascular temperature sensor (40); and
a wire lumen (593) comprising electrical wirings (560) for the thermocouple or thermistor and the ISFET pH sensor (30).

15. The system according to any one of claims 1 to 4, wherein the SO₂ sensor (20) is a pulse oximeter.
